# EUROPEAN PATENT APPLICATION

(11) **EP 4 012 414 A1**
(43) Date of publication of application: **15.06.2022**
(21) Application number: 20213627.1
(22) Date of filing: 11.12.2020
(51) Int. Cl.: G01N 33/68

(54) **DIAGNOSIS OF AUTISM SPECTRUM DISORDER PHENOTYPE 1**

(71) Applicant: Stalicla S.A., 1202 Geneva (CH)
(72) Inventor: DURHAM, Lynn, 1202 Geneva (CH)
(74) Representative: Stolmár & Partner Intellectual Property GmbH

(57) **Abstract**

The invention relates to a method for diagnosing ASD, comprising a) providing a sample from a patient; b) measuring the level of at least one metabolic marker selected from the group consisting of ribitol, lyxonate, erythritol, ribose and urate in said sample; and c) diagnosing ASD if the level of the at least one metabolic marker is specifically different in comparison to a typically developing control.

## Description

### Field of the invention

The invention relates to methods of diagnosing autism spectrum disorder (ASD), as well as to methods for monitoring ASD progression.

### Background of the invention

ASD is one of the most prevalent and disabling neurodevelopmental disorder. The prevalence of ASD is currently estimated at 1% in the world and 1 in 59 school-aged children in the US (1 in 37 boys and 1 in 151 girls) (Baio et al. Prevalence of Autism Spectrum Disorder Among Children Aged 8 Years - Autism and Developmental Disabilities Monitoring Nertwork, 11 sites, United States, MMWR. Surveillance Summaries 67, no. 6: 1-23)

ASD is currently considered a single diagnostic entity characterized by 1) deficits in social interactions and communication, including deficits in social-emotional reciprocity, deficits in nonverbal communicative behavior used for social interaction, and deficits in developing, maintaining, and understanding relationships; 2) at least 4 subdomains of restricted or repetitive behaviors, including stereotyped or repetitive motor movements, insistence on sameness or inflexible adherence to routines, highly restricted, fixated interests, hyper- or hyporeactivity to sensory input, or unusual interest in the sensory aspects of the environment (Baird G, et al. Neurodevelopmental disorders. American Psychiatric Association. Diagnostic and Statistical Manual of Mental Disorders-Fifth Edition (DSM-5). Washington, D.C.: American Psychiatric Publishing, 2013: p. 31-86).

Early manifestations of core symptoms can be observed as early as 9 to 12 months (Roger SL et al. Autism treatment in the first year of life: a pilot study of infant start, a parent-implemented intervention for symptomatic infants. J Autism Dev Disord. 2014;44(12):2981-95) and a stable diagnosis can be established as early as in the 14^{th} month (Pierce K et al. Evaluation of the Diagnostic Stability of the Early Autism Spectrum Disorder Phenotype in the General Population Starting at 12 Months: JAMA Pediatr. 2019;173(6):578-587).

However, the core symptoms may not become fully manifest until social demands exceed limited capacities or may be masked by learner strategies in later life (Baird G. Classification of Diseases and the Neurodevelopmental Disorders: The Challenge for DSM-5 and ICD-11. Developmental Medicine & Child Neurology. 2013;55(3):200-201). For ASD to be diagnosed, its manifestations must cause clinically significant impairment affecting the ability of patients to interact with others, especially people of their own age when referring to pediatric patients. This means that using classical diagnosis, medical intervention is only possible once symptoms have started to emerge.

As the underlying causes of ASDs remain elusive, attempts have been made to stratify ASD patients into smaller, more homogeneous subgroups by utilizing specific genetic signatures (Bernier et al; Disruptive CHD8 mutations define a subtype of autism early in development; Cell 2014 Jul 17; 158 (2): 263-276.) or behavioral and clinical endophenotypes (Eapen V. and Clarke R.A.; Autism Spectrum Disorders: From genotypes to phenotypes; Front Hum Neurosci. 2014;8:914). However, these strategies face difficulty encompassing the genetic and phenotypic heterogeneity of ASD and may not assist in the identification of specific neurobiological pathways underlying disease.

Assays on a molecular basis might provide a way to classify ASD patients. However, because of the intrinsic complexity of ASD, its heterogeneity and the complex intertwining of genetic and environmental causal factors, specific biomarkers for ASD which could be used to establish such an assay have yet to be identified. Moreover, because of their specificity, such biomarkers cannot encompass large groups of ASD patients. Such assays could however in the short term come to support the characterization of genotypically, phenotypically or treatment response pre-identified subgroups.

Previously, methods directed to identifying a subset of idiopathic autism spectrum disorder, so called ASD phenotype 1, has been reported. This subset of patients can be identified according to the co-occurrence of clinical signs and symptoms. Beside these clinical signs and symptoms, ASD phenoytpe 1 can be identified as described in PCT/EP2018/080372 and PCT/EP2019/080450, by administering sulforaphane, an Nrf2 activator, to an ASD patient or to lymphoblastoid cell lines (LCL) derived from the blood of this patient, respectively, and identifying the patient as ASD phenotype 1 if the patient show a negative behavioral response or if the patient's cells do not show decrease in production of energy after administration of the Nrf2-activator. However, the *in vivo* nature of the method described in PCT/EP2018/080372 greatly limits its clinical development. As for the method described in PCT/EP2019/080450, generation of LCLs generation from patient blood and subsequent *in vitro* testing is a long and complex procedure, limiting its clinical translatability. A laboratory test performed on unprocessed biosample from patients, such as blood, urine, or biosamples that require minimal technical preparation, such as plasma, would be of great advantage to circumvent the limitation of the *in vivo* and *in vitro* testing methods known in the art.

Metabolomic analysis of body specimens (i.e., plasma, serum, urine) has been recently utilized to further characterize the pathogenic mechanisms of several complex disorders, including ASD (Kuwabara, H. et al. Altered metabolites in the plasma of autism spectrum disorder: a capillary electrophoresis time-of-flight mass spectroscopy study. PloS One 8, e73814 (2013); West, P. R. et al. Metabolomics as a tool for discovery of biomarkers of autism spectrum disorder in the blood plasma of children. PloS One 9, e112445 (2014); Wang, H. et al. Potential serum biomarkers from a metabolomics study of autism. J. Psychiatry Neurosci. JPN 41, 27-37 (2016); Delaye, J.-B. et al. Post hoc analysis of plasma amino acid profiles: towards a specific pattern in autism spectrum disorder and intellectual disability. Ann. Clin. Biochem. 55, 543-552 (2018); Rangel-Huerta, O. D. et al. Metabolic profiling in children with autism spectrum disorder with and without mental regression: preliminary results from a cross-sectional case-control study. Metabolomics Off. J. Metabolomic Soc. 15, 99 (2019); Orozco, J. S., Hertz-Picciotto, I., Abbeduto, L. & Slupsky, C. M. Metabolomics analysis of children with autism, idiopathic-developmental delays, and Down syndrome. Transl. Psychiatry 9, 243 (2019); Smith, A. M. et al. A Metabolomics Approach to Screening for Autism Risk in the Children's Autism Metabolome Project. Autism Res. Off. J. Int. Soc. Autism Res. (2020); Yang, J. et al. Assessing the Causal Effects of Human Serum Metabolites on 5 Major Psychiatric Disorders. Schizophr. Bull. 46, 804-813 (2020)). Moreover, metabolic analysis of urine has been recently utilized to monitor the efficacy of pharmacological treatment for ASD (Bent, S. et al. Identification of urinary metabolites that correlate with clinical improvements in children with autism treated with sulforaphane from broccoli. Mol. Autism 9, 35 (2018)). In this regard, several groups proposed methods to improve diagnosis of autism and/or offer earlier diagnosis based on the determination of specific metabolites, such as 4-ethylphenylsulfate, indolepyruvate, glycolate, or imidazole proprionate (Hsaio et al., US20140065132A1), a plurality of metabolites having a molecular weight from about 10 Daltons to about 1500 Daltons (Gebrin Cezar et al. EP2564194A1), 12-HETE, 15-HETE and sphingosine/choline (Srivastava et al. US20170067884A1). Other proposed methods detect differences in expression, such as expression of a carbohydrate metabolic enzyme protein (Lipkin et al. US20120207726A1) or expression of the Gc globulin protein (Horning et al. WO20133130953A2).

There is therefore a need for an efficient and easy laboratory method for diagnosing ASD patients, who could benefit from targeted pharmaceutical intervention addressing the underlying molecular dysfunction of their ASD subgroup.

### Objective problem to be solved

The problem to be solved is the provision of means to efficiently identify a specific subgroup of ASD patients, so called ASD Phenotype 1.

Another problem to be solved is to efficiently monitor disease severity, disease severity improvement or monitoring of pharmacological or therapeutic treatment efficacy using biological markers specific for the patients in this subgroup.

### Summary of the invention

The problem is solved by a method for diagnosing ASD, comprising
a) providing a sample from a patient;
b) measuring the level of at least one metabolic marker selected from the group consisting of ribitol, lyxonate, erythritol, ribose and urate in said sample;
c) diagnosing ASD if the level of the at least one metabolic marker is specifically different in comparison to a typically developing control.

The problem is further solved by a method for diagnosing ASD, comprising
a) providing a sample from a subject;
b) measuring at least one metabolite ratio selected from the group consisting of ribitol/creatinine, ribitol/1,5-anhydroglucitol, ribitol/deoxycarnitine, urate/N-acetylcarnosine, erythritol/1,5-anyhdroglucitol and erythrithol/N-acetylcarnosine in said sample;
c) diagnosing ASD if the at least one metabolite ratio measured in step b) is increased in comparison to a typically developing control.

The problem is also solved by a method for monitoring the progression of ASD in a patient suffering from ASD, comprising
a) providing a first sample from the patient taken at time point t₀;
b) measuring the level of at least one metabolic marker selected from the group consisting of ribitol, lyxonate, erythritol, ribose and urate in said first sample;
c) providing a second sample from the patient taken at time point t₁;
d) measuring the level of the same metabolic marker as in step b);
e) determining whether the ASD has progressed in the patient by comparing the level obtained in step b) with the one obtained in step d).

The problem is also solved by a method for monitoring the progression of ASD in a patient suffering from ASD, comprising
a) providing a first sample s₀ from the patient;
b) measuring at least one metabolite ratio selected from the group consisting of ribitol/creatinine, ribitol/1,5-anhydroglucitol, ribitol/deoxycarnitine, urate/N-acetylcarnosine, erythritol/1,5-anyhdroglucitol and erythrithol/N-acetylcarnosine in said first sample;
c) providing a second sample s₁ from the patient;
d) measuring the same metabolite ratio selected from the group consisting of ribitol/creatinine, ribitol/1,5-anhydroglucitol, ribitol/deoxycarnitine, urate/N-acetylcarnosine, erythritol/1,5-anyhdroglucitol and erythrithol/N-acetylcarnosine as in step b) in said second sample;
e) determining whether the ASD has progressed in the patient by comparing the ratios measured in step b) with the one obtained in step d).

The problem is also solved by a method for determining efficacy of an ASD treatment in an ASD patient, comprising:
a) providing samples from the patient obtained before and after application of the medical intervention;
b) measuring at least one metabolite ratios selected from the group consisting of ribitol/creatinine, ribito/1,5-anhydroglucitol, ribitol/deoxycarnitine, urate/N-acetylcarnosine, erythritol/1,5-anyhdroglucitol and erythrithol/N-acetylcarnosine in the samples of step a).

### Brief description of the figures

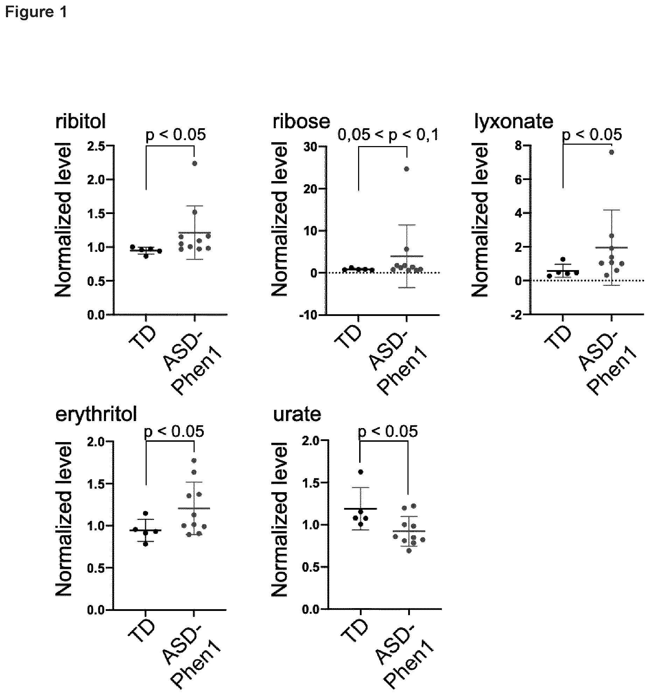

Figure 1: shows a graphic representation of levels of metabolites found to be specifically different in plasma samples from ASD Phenotype 1 compared to TD individuals. Data are represented as individual values, mean ± s.d. 1,5-AG: 1,5-anhydroglucitol; NAC: N-acetylcarnosine. N = 5 for TD and N = 10 for ASD-Phen1.

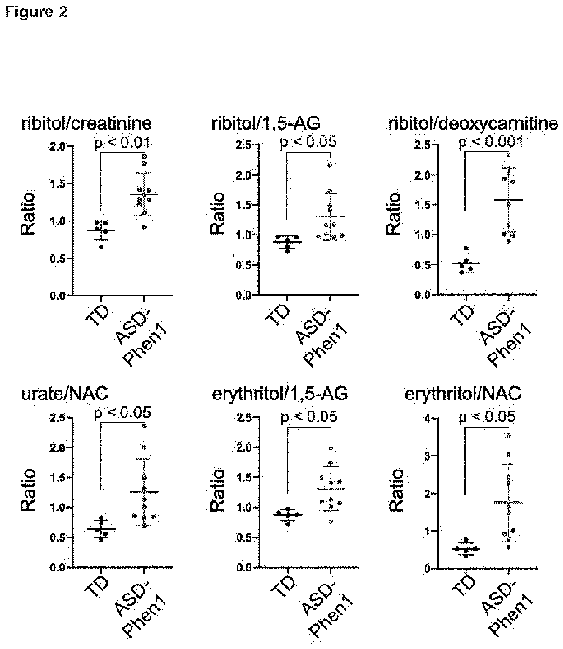

Figure 2: shows a graphic representation of the ratio between selected metabolites. Data are represented as individual values, mean ± s.d. 1,5-AG: 1,5-anhydroglucitol; NAC: N-acetylcarnosine. N = 5 for TD and N = 10 for ASD-Phen1.

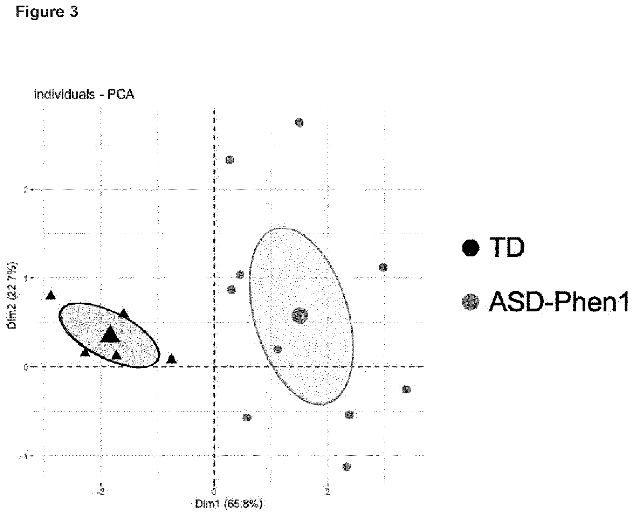

Figure 3: Biplot representation of dimension 1 and dimension 2 of the Principal Component Analysis (PCA). Data are represented as individual datapoint, average ± s.d. N = 5 for TD and N = 10 for ASD-Phen1.

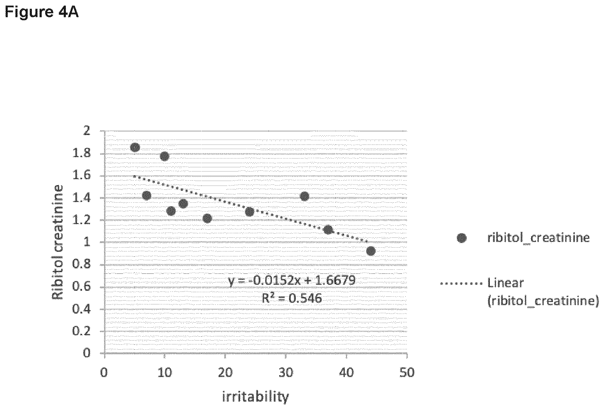

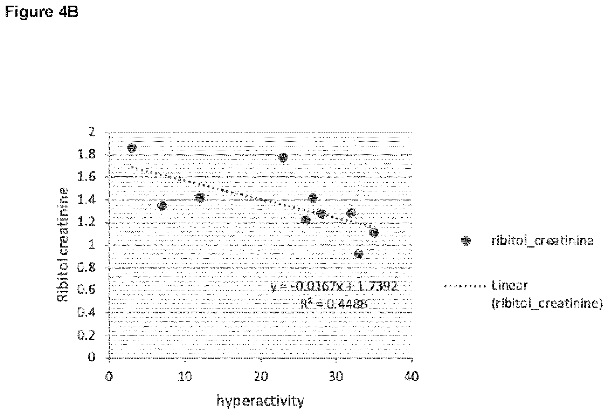

Figure 4: A. shows a graphic representation of the correlation between ribitol/creatinine ratio and the ABC irritability subscale. N = 10 patients with ASD Phenotype 1. B. graphic representation of the correlation between ribitol/creatinine ratio and the ABC hyperactivity subscale. N = 10 patients with ASD Phenotype 1.

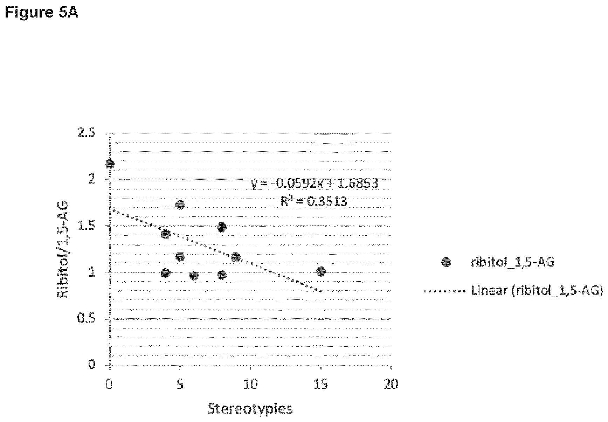

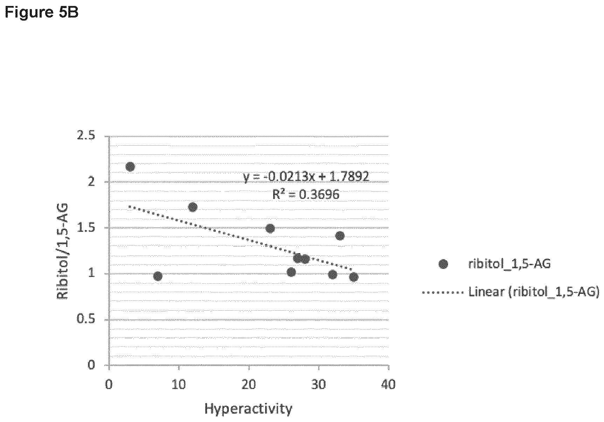

Figure 5: A. shows a graphic representation of the correlation between ribitol/1,5-anhydroglucitol (1,5-AG) ratio and the ABC stereotypies subscale. N = 10 patients with ASD Phenotype 1. B. graphic representation of the correlation between ribitol/1,5-anhydroglucitol (1,5-AG) ratio and the ABC hyperactivity subscale. N = 10 patients with ASD Phenotype 1.

### Detailed description of the invention

In a first aspect, the invention relates to a method for diagnosing autism spectrum disorder (ASD) in a subject, comprising
a) providing a sample from a patient;
b) measuring the level of at least one metabolic marker selected from the group consisting of ribitol, lyxonate, erythritol, ribose and urate in said sample;
c) diagnosing ASD if the level of the at least one metabolic marker is specifically different in comparison to a typically developing control.

As used herein, the term autism spectrum disorder (ASD) is understood to cover a family of neurodevelopmental disorders characterized by deficits in social communication and interaction and restricted, repetitive patterns of behavior, interests or activities.

The term "ASD patient" refers to a patient having received a formal diagnosis of ASD. The term "subject" herein refers to humans suspected of having ASD, i.e. subjects presenting behavioral characteristics of ASD and displaying clinical signs of ASD but who have not yet received a formal validation of their diagnostic.

The person skilled in the art is well aware of how a patient may be diagnosed with ASD, in particular idiopathic ASD. For example, the skilled person may follow the criteria set up in "American Psychiatric Association; Diagnostic and Statistical Manual of Mental Disorders (DSM-5) Fifth edition" to give a subject a diagnosis of ASD. Likewise, ASD patients may have been diagnosed according to standardized assessments tools including but not limited to DSM IV, ICD-9, ICD-10, DISCO, ADI-R, ADOS, CHAT. In other cases, patients may have a well-established DSM-IV diagnosis of autistic disorder, or pervasive developmental disorder not otherwise specified (PDD-NOS).

Herein, the term "typically developing individual (TD)" refers to a subject that has neither been diagnosed with ASD nor shows any clinical signs and symptoms of ASD.

The method according to the invention provides for the first time a laboratory test performed on a sample derived from a subject for diagnosing ASD, in particular ASD phenotype 1. Unlike assessment tools known in the art, the methods of the invention can be carried out on a sample of the patient, without the need for the patient to be present during assessment. In addition, the methods of the invention do not require the presence of a medical doctor or physician, but can be carried out by a laboratory assistant in an automatized fashion. This not only reduces costs, but also enhances reliability of the diagnosis.

The methods of the invention comprise a step of providing a sample from a subject or patient. The sample may be any suitable biological sample such as blood, saliva, urine, feces or cerebrospinal fluid. Samples that can be used in the methods of the invention are easily obtainable and do not require surgery, which is a particular advantage when working with subjects who are likely to exhibit some degree of social impairment.

In a preferred embodiment, the sample is a blood sample such as a plasma sample or a serum sample. The person skilled in the art knows how plasma can be isolated from the blood of a subject. The blood sample may be peripheral blood or a whole-blood sample that has been processed, e.g. by purification or separation of individual compounds or urine or cerebrospinal fluid.

The sample may be purified or prepared in order to facilitate subsequent steps. In particular, the sample may be prepared by removing proteins and otherwise purifiying the sample. After preparation, the sample may be either processed immediately or kept at -70°C until further analyses.

The methods of the invention further comprise a step of measuring the level of at least one metabolite marker selected from the group consisting of ribitol, lyxonate, erythritol, ribose and urate in said sample in the sample.

Ribitol (Human Metabolome Database (HMDB) identification numbers: HMDB0002917, HMDB0000508, HMDB0001851, HMDB0000568), also referred to as adonitol, is a crystalline pentose alcohol (C₅H₁₂O₅) formed by the reduction of ribose having the structure of chemical formula (I):

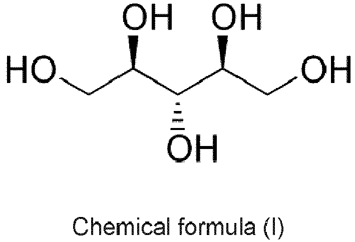

Lyxonate, also referred to as 2,3,4,5-tetrahydroxypentanoic acid, has the structure of chemical formula (II)

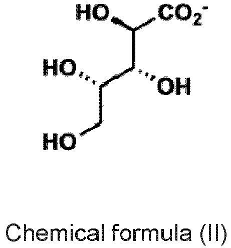

Erythritol (HMDB identification number: HMDB0002994), also refered as (2R,3S)-butane-1,2,3,4-tetrol (C₄H₁₀O₄), belongs to the class of sugar alcohols and has the structure of chemical formula (III):

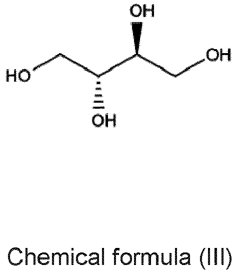

D-Ribose (HMDB identification number: HMDB0000283), commonly referred to simply as ribose or alternatively as D-ribofuranoside or (3R,4S,5R)-5-(hydroxymethyl)oxolane-2,3,4-triol (C₅H₁₀O₅), is a five-carbon sugar belonging to the class of pentoses. It has the structure of chemical formula (IV):

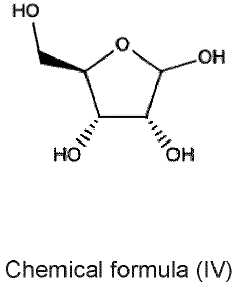

Urate (HMBD identification number: HMDB0000289), also referred to as uric acid or alternatively as 2,3,6,7,8,9-hexahydro-1H-purine-2,6,8-trione (C₅H₄N₄O₃), is a purine derivative of the class of xanthines with a ketone group conjugated at carbons 2 and 6 of the purine moiety. It has the structure of chemical formula (V):

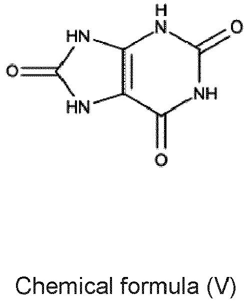

The person skilled in the art is well aware of how to assess the level of a metabolite marker in a sample.

Detecting the levels of metabolites in the samples can be performed by any method known in the art, e.g. by liquid chromatography tandem mass spectroscopy (Wamelink M.M.C et al. Quantification of sugar phosphate intermediates of the pentose phosphate pathway by LC-MS/MS: application to two new inherited defects of metabolism, J Chromatogr B Analyt Technol Biomed Life Sci. 823(1): 18-25 (2005)). Alternative methods include colorimetric assays (Novello F. et al. The pentose phosphate pathway of glucose metabolism. Measurement of the non-oxidative reactions of the cycle, Biochem J. 107(6):775-91 (1968)), chromatography using ¹⁴C-labelled substrates (Becker M.A. Patterns of phosphoribosylpyrophosphate and ribose-5-phosphate concentration and generation in fibroblasts from patients with gout and purine overproduction, J Clin Invest. 57(2):308-18 (1976)), capillary electrophoresis coupled with mass spectroscopy (Soga T. Capillary electrophoresis-mass spectrometry for metabolomics, Methods Mol Biol 358:129-3 (2007)), gas chromatography coupled with mass spectroscopy (Koek M.M. et al. Microbial metabolomics with gas chromatography/mass spectrometry, Analytical Chemistry, 15;78(4):1272-81 (2006)) or nuclear magnetic resonance (Ardenkjaer-Larsen J.H. et al. Increase in signal-to-noise ratio of > 10,000 times in liquid-state NMR. PNAS 100(18):10158-63 (2003)).

In a preferred embodiment, reverse phase/ultrahigh performance liquid chromatography-tandem mass spectroscopy methods with positive ion mode electrospray ionization (ESI) or reverse phase/ultrahigh performance liquid chromatography-tandem mass spectroscopy with negative ion mode ESI is used.

In one embodiment, identification of the peak corresponding to each metabolites is based on retention time/index (RI), mass to charge ratio (m/z), and chromatographic data (including MS/MS spectral data). The identity of the metabolites is confirmed by comparing with an existing library of authenticated standards. A metabolite identity is confirmed when retention index is within a narrow RI window of the proposed identification, accurate mass match to the library +/- 10 particle per million, and the MS/MS forward and reverse scores between the experimental data and authentic standards. Each metabolite is characterized by a specific peak in these methods and level of the metabolite corresponds to the area-under-the-curve of these peaks.

In step c) of the method according to the invention, the level of the at least one metabolic marker is compared to the level of the same metabolic marker in a sample from a typically developing control and ASD is diagnosed if the level of the metabolic marker in the subject is specifically different from the level in the typically developing control.

A sample from a typically developing control may be a sample from a specific individual or a pool of several samples taken from several different individuals. Preferably, the typically developing control is age-matched and sex-matched to the subject.

According to the invention, specifically different means that in case the metabolic marker is ribitol, lyxonate, erythritol or ribose, the level in the sample from the subject is higher than in a typically developing control and in case the metabolic marker is urate, the level in the sample from the subject is lower than in a typically developing control.

In one embodiment, "higher" means an increase of at least 15 percent, while "lower" means a decrease of at least 15 percent.

In a second aspect, the invention is directed to a method for diagnosing ASD in a subject, comprising
a) providing a sample from a subject;
b) measuring at least one metabolite ratio selected from the group consisting of ribitol/creatinine, ribitol/1,5-anhydroglucitol, ribitol/deoxycarnitine, urate/N-acetylcarnosine, erythritol/1,5-anyhdroglucitol and erythrithol/N-acetylcarnosine in said sample;
c) diagnosing ASD if the at least one metabolite ratio measured in step b) is increased in comparison to a typically developing control.

In the step of measuring at least one metabolite ratio, the level of the first and second metabolite are measured and then optionally converted into the same unit. Finally, the ratio between the levels is obtained by dividing the first level by the second level.

According to the invention, the at least one metabolite ratio is selected from the group consisting of ribitol/creatinine, ribitol/1,5-anhydroglucitol, ribitol/deoxycarnitine, urate/N-acetylcarnosine, erythritol/1,5-anyhdroglucitol and erythrithol/N-acetylcarnosine.

Creatinine (HMDB identification number: HMDB0000562), also referred to as creatine anhydride or alternatively as 2-imino-1-methylimidazolidin-4-one (C₄H₇N₃O), belongs to the class of alpha amino acids and derivatives and is a breakdown product of creatine phosphate. It has the structure of chemical formula (VI):

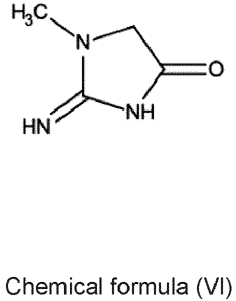

1,5-anhydroglucitol or 1,5-anhydrosorbitol (HMDB identification number: HMDB0002712), also referred to as (2R,3S,4R,5S)-2-(hydroxymethyl)oxane-3,4,5-triol (C₆H₁₂O₅), belongs to the class of monosaccharides. It has the structure of chemical formula (VII):

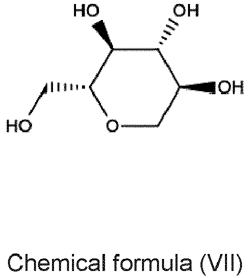

Deoxycarnitine (HMBD identification number: HMDB0001161), also referred to as 3-dehydroxycarnitine or 4-Trimethylammoniobutanoic acid or gamma-butyrobetaine or 4-(trimethylazaniumyl)butanoate (C₇H₁₅NO₂), belongs to the class of straight chain fatty acids. It has the structure of chemical formula (VIII):

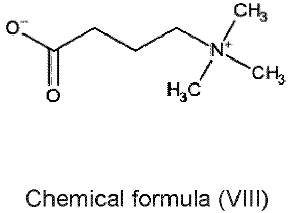

N-Acetylcarnosine (HMDB identification number: HMDB0012881), also referred to as (2R)-2-(3-acetamidopropanamido)-3-(3H-imidazol-4-yl)propanoic acid (C₁₁H₁₆N₄O₄) belongs to the class of organic compounds known as hybrid peptides. It has the structure of chemical formula (IX):

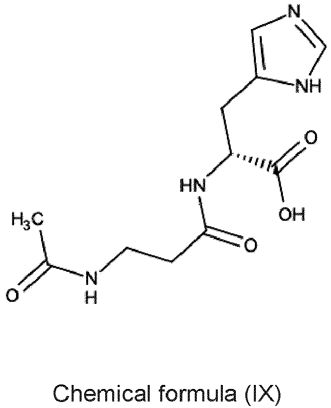

The inventors have surprisingly found that an increase in the metabolite ratios ribitol/creatinine, ribitol/1,5-anhydroglucitol, ribitol/deoxycarnitine, urate/N-acetylcarnosine, erythritol/1,5-anyhdroglucitol and erythrithol/N-acetylcarnosine is associated with a diagnosis of ASD.

Without wanting to be bound by a mechanism, it is believed that a subtype of ASD patients exhibit an increased activity of the pentose phosphate pathway. This is further described in WO2020094748 A1. As a consequence, these patients are expected to have increased levels of metabolites related to this pathway, namely ribitol, lyxonate, erythritol, D-ribose and urate. In contrast thereto, the levels of creatinine, 1,5-anhydroglucitol, deoxycarnitine or N-acetylcarnosine are not expected to be increased in these patients or may even be decreased, as observed in other populations of patients with NDD (Neul, J. L. et al. Metabolic Signatures Differentiate Rett Syndrome From Unaffected Siblings. Front. Integr. Neurosci. 14, 7 (2020)). The ratios ribitol/creatinine, ribitol/1,5-anhydroglucitol, ribitol/deoxycarnitine, urate/N-acetylcarnosine, erythritol/1,5-anyhdroglucitol and erythrithol/N-acetylcarnosine are therefore expected to be higher in ASD patients compared to typically developing individuals.

In one embodiment, the increase of the at least one metabolite ratio is at least 15 percent, preferably 30 percent, most preferably 50 percentage increased over the respective metabolite ratio in a typically developing control.

In one embodiment, 2, preferably 4, most preferably 6 metabolite ratios are measured. The more metabolite ratios are measured, the more reliable the diagnosis is.

If more than one metabolite ratio is measured, an increase of the metabolite ratios is measured by calculating the mean value of the metabolite ratios measured in the sample and comparing it to the mean value of the respective metabolite ratios measured in a typically developing control. In one embodiment, ASD is diagnosed if this increase is at least 15 percent, preferably 30 percent, most preferably 50 percentage of the mean value of the respective metabolite ratios measured in a typically developing control.

In one embodiment, the subject is further diagnosed as a subtype of ASD termed ASD phenotype 1. The subject being diagnosed as ASD phenotype 1 may have been previously diagnosed with idiopathic ASD. In the following, the terms "idiopathic autism spectrum disorder", "idiopathic autism" and "idiopathic ASD" and the terms "ASD phenotype 1", "phenotype 1" and "ASD-Phen1" are used interchangeably.

Causal genetic factors can only be identified in 15 to 20% of patients with ASD symptoms. The diagnosis of "idiopathic ASD" that is applied to the vast majority of ASD patients is therefore a behavioral umbrella term for a large group of neurodevelopmental disorders, which actually differ significantly in their molecular and genetic with different etiologies.

There is growing perception among the scientific community that this approach should be replaced by classification of patients allowing for targeted therapy. But although recent developments of new genetic screening methods have permitted to detect hundreds of genetic risk factors, including common and rare genetic variants, which can increase the likelihood of ASD (Ronemus M. et al; The role of the novo mutations in the genetics of autism spectrum disorders; Nat Rev Genet. 2014 Feb; 15(2): 133-41), none of these individual risk factors is, in itself, sufficiently significant to allow for a clear diagnosis of ASD.

It has, however, been hypothesized that the ever-expanding number of ASD susceptibility genes converge towards a limited number of molecular pathways. Identification of these pathways would offer exciting chances both for diagnosis and treatment, which could then be based on and targeted to actual molecular and genetic causes, rather than being symptom based.

In contrast to the umbrella definition of idiopathic ASD, ASD phenotype 1 patients represent a molecularly and genetically defined subset of ASD patients characterized by an upregulation of pathways involved in adaptation to stress, apoptosis or cell differentiation, cell proliferation, cell cycle progression, cell division and differentiation (in particular but not limited to PI3K, AKT, mTOR, MAPK, ERK/JNK-P38). Proinflammatory cytokines (TNF-a, IL-6, IL-1β, IL-17A, IL-22 and GM-CSF), Th1 cytokine (INF-γ) and chemokine (IL-8) may also be significantly increased in the brain of these patients compared to healthy control patients.

ASD phenotype 1 patients may be further characterized by:
- presence of at least 1 of the following two mandatory clinical signs and symptoms:
   ∘ Enlarged head size versus control population characterized by at least one standard deviations above the mean head circumference (HC) during the first 24 months of life and/or formal macrocephaly (HC>97% of the general population); and /or
   ∘ Cyclic aggravation of core symptoms potentiated by periods of infectious events, deciduous tooth loss, post-traumatic injury, endogenous and exogenous temperature variation.
   and
- presence of at least 2, and most preferably at least 3 out of the following 20 characteristics:
   ∘ Accelerated hair and nail growth versus control population
   ∘ Increased tendency to present with lighter colors of skin and eyes as compared to individuals of the same ethnicity
   ∘ Substantially longer eyelashes than control subjects of the same ethnicity
   ∘ At least 5 non-contiguous areas of hypopigments skin, particularly presenting on the back of the patient
   ∘ Signs of edema during periods of infections events, deciduous tooth loss, post-traumatic injury, or endogenous and exogenous factors modifying body temperature; more specifically, facial edema locate in the periorbital and forehead areas
   ∘ Increased blood levels of gamma-glutamyl transpeptidase (GGT) as compared to typically developing individuals of the same ethnicity.
   ∘ Congenital genitourinary malformations and/or functional impairment to initiate urinating
   ∘ Hypoplasia of the patella
   ∘ Frequent episodes of diarrhea specifically before the age of 5 years
   ∘ Frequent episodes of tinnitus
   ∘ Thinning or absence of the corpus callosum
   ∘ Positive family history for hematological malignancies in particular but not limited to myeloma and acute promyelocytic leukemia
   ∘ Positive family history for rheumatoid arthritis, that is at least two affected first-degree relatives in two consecutive generations
   ∘ Adverse events in response to acetyl-salicylic acid or its derivatives
   ∘ Iris coloboma, either monoliteral or bilateral
   ∘ Sleep hyperhidrosis particularly as babies, toddlers and young children (notably increased night sweating during infancy and childhood - often reported by relatives to require bed linen changes)
   ∘ Increased Th1/Th2 ratio (i.e. elevated levels of interleukin 1 beta, interleukin 6, TNF-alpha, interferon gamma)
   ∘ Congenital accessory or duplicated spleen
   ∘ Congenital absence of cisterna chyli
   ∘ Delayed tooth growth
   ∘ Reported history of mother suffering viral or bacterial infection during pregnancy and/or biologically confirmed maternal immune activation during pregnancy

Because ASD phenotype 1 patients share a common molecular pathology, identifying a patient as ASD phenotype 1 enables the selection of an adequate treatment targeting the underlying genetic causes, instead of only treating behavioral symptoms. Treatments that are specific for ASD phenotype 1 patients have been previously described (EP 3498297 A1). The methods of invention thus allow to select patients that will benefit from such specific treatments.

In a third aspect, the invention relates to a method for monitoring the progression of ASD in a patient suffering from ASD, comprising
a) providing a first sample from the patient taken at time point t₀;
b) measuring the level of at least one metabolic marker selected from the group consisting of ribitol, lyxonate, erythritol, ribose and urate in said first sample;
c) providing a second sample from the patient taken at time point t₁;
d) measuring the level of the same metabolic marker as in step b);
e) determining whether the ASD has progressed in the patient by comparing the level obtained in step b) with the one obtained in step d).

The method comprises measuring the level of a metabolite marker in two different samples, a first sample s₀ and a second sample s₁. Preferably, the first and the second sample are the same kind of sample, i.e. both are blood samples such as plasma samples or both are urine samples.

According to the invention, the first sample is obtained at a time point t₀ and the second sample is obtained at a later time point t₁. Between t₀ and t₁, different events may take place. For example, the patient may receive a medical intervention.

A medical intervention is herein defined as an activity directed at or performed on an individual with the object of improving health and treating disease. Examples of medical interventions include administration of a drug or drug combination, application of a gene therapy or behavioural therapy.

In case a medical intervention takes place between t₀ and t₁, the method is suitable for determining whether the treatment is effective in the patient. t₁ may also be chosen so that between t₀ and t₁ a certain amount of time has lapsed, i.e., one month, three months, six months or a year. In this case, the method is useful for monitoring disease progression over the certain amount of time.

In step e) of the method, the levels of the at least metabolite marker obtained for sample s₀ and s₁ are compared with each other. In one embodiment, it is determined that the ASD has progressed in the patient if, in case the at least one metabolic marker is ribitol, lyxonate, erythritol or ribose, the level in the second sample is higher than the level in the first sample or if, in case the at least one metabolic marker is urate, the level in the second sample is lower than the level in the first sample.

In a fourth aspect, the invention relates to a method for monitoring the progression of ASD in a patient suffering from ASD, comprising
a) providing a first sample s₀ from the patient;
b) measuring at least one metabolite ratio selected from the group consisting of ribitol/creatinine, ribitol/1,5-anhydroglucitol, ribitol/deoxycarnitine, urate/N-acetylcarnosine, erythritol/1,5-anyhdroglucitol and erythrithol/N-acetylcarnosine in said first sample;
c) providing a second sample s₁ from the patient;
d) measuring the same metabolite ratio selected from the group consisting of ribitol/creatinine, ribitol/1,5-anhydroglucitol, ribitol/deoxycarnitine, urate/N-acetylcarnosine, erythritol/1,5-anyhdroglucitol and erythrithol/N-acetylcarnosine as in step b) in said second sample;
e) determining whether the ASD has progressed in the patient by comparing the ratios measured in step b) with the one obtained in step d).

According to the invention, the first sample s₀ is obtained at a time point t₀ and the second sample s₁ is obtained at a later time point t₁. Between t₀ and t₁, different events may take place. For example, the patient may receive a medical intervention.

In case the patient receives a medical intervention between t₀ and t₁, the method is suitable for determining whether the treatment is effective in the patient. t₁ may also be chosen so that between t₀ and t₁ a certain amount of time has lapsed, i.e., one month, three months, six months or a year. In this case, the method is useful for monitoring disease progression in the patient over the certain amount of time.

In step e) of the method, the at least one metabolite ratio obtained for sample s₀ and s₁ are compared with each other. In one embodiment, it is determined that the ASD has progressed in the patient if the ratio measured in the second sample is higher than the corresponding ratio measured in the first sample.

In one embodiment, 2, 4 or 6 ratios for each sample are measured, and the mean value of these ratios is calculated for each sample. These mean values are used in step e) instead of the ratios for determining whether the ASD has progressed. The use of the mean value of several different metabolite ratios further improves the predictive value of the method of the invention.

In a fifth aspect, the invention is directed to a method for determining efficacy of an ASD treatment in an ASD patient, comprising:
a) providing samples from the patient obtained before and after application of the medical intervention;
b) measuring at least one metabolite ratios selected from the group consisting of ribitol/creatinine, ribito/1,5-anhydroglucitol, ribitol/deoxycarnitine, urate/N-acetylcarnosine, erythritol/1,5-anyhdroglucitol and erythrithol/N-acetylcarnosine in the samples of step a).

The method according to the invention is useful for assessing the response of a patient to a certain medical intervention. Said medical intervention may be an established treatment, so that the method is used to determine whether the particular medical intervention shows efficacy in improving the specific patient's ASD progression. The medical intervention may also be a novel treatment that has not been tested yet on patients on a large scale. In this case, the method is useful for determining efficacy of the medical intervention as such.

### Examples

### Example 1

### Materials & methods

Prior to metabolomic characterization of plasma samples from ASD phenotype 1 patients, patients were classified as ASD phenotype 1 or controls.

Individuals with idiopathic ASD were classified as ASD phenotype 1 if they showed:
- At least 1 mandatory criterion:
   ∘ Enlarged head size versus control population characterized by at least one standard deviation above the mean head circumference (HC) during the first 24 months of life and/or formal macrocephaly (HC>97% of the general population).
      And/or
   ∘ Cyclic aggravation of core symptoms potentiated by periods of infectious events, deciduous tooth loss, post-traumatic injury, endogenous and exogenous temperature variation.
   And
- At least 2, and most preferably at least 3 out of the following 20 characteristics:
   ∘ Accelerated hair and nail growth versus control population
   ∘ Increased tendency to present with lighter colors of skin and eyes as compared to individuals of the same ethnicity
   ∘ Substantially longer eyelashes than control subjects of the same ethnicity
   ∘ At least 5 non-contiguous areas of hypopigments skin, particularly presenting on the back of the patient
   ∘ Signs of edema during periods of infections events, deciduous tooth loss, post-traumatic injury, or endogenous and exogenous factors modifying body temperature; more specifically, facial edema locate in the periorbital and forehead areas
   ∘ Increased blood levels of gamma-glutamyl transpeptidase (GGT) as compared to typically developing individuals of the same ethnicity.
   ∘ Congenital genitourinary malformations and/or functional impairment to initiate urinating
   ∘ Hypoplasia of the patella
   ∘ Frequent episodes of diarrhea specifically before the age of 5 years
   ∘ Frequent episodes of tinnitus
   ∘ Thinning or absence of the corpus callosum
   ∘ Positive family history for hematological malignancies in particular but not limited to myeloma and acute promyelocytic leukemia
   ∘ Positive family history for rheumatoid arthritis, that is at least two affected first-degree relatives in two consecutive generations
   ∘ Adverse events in response to acetyl-salicylic acid or its derivatives
   ∘ Iris coloboma, either monoliteral or bilateral
   ∘ Sleep hyperhidrosis particularly as babies, toddlers and young children (notably increased night sweating during infancy and childhood - often reported by relatives to require bed linen changes)
   ∘ Increased Th1/Th2 ratio (i.e. elevated levels of interleukin 1 beta, interleukin 6, TNF-alpha, interferon gamma)
   ∘ Congenital accessory or duplicated spleen
   ∘ Congenital absence of cisterna chyli
   ∘ Delayed tooth growth
   ∘ Reported history of mother suffering viral or bacterial infection during pregnancy and/or biologically confirmed maternal immune activation during pregnancy

Control patients were identified as individuals in which no signs or symptoms of neurobehavioral disorders have been detected and are therefore considered as typically developing individuals (TD).

The data reported in the present patent were generated from plasma isolated from peripheral blood. Tubes containing anti-coagulant heparin were used to collect blood samples via venipuncture. Within 30-40 minutes of collection, plasma was isolated by centrifugation of whole blood for 15 minutes at room temperature in a swinging bucket rotor.

Samples to be analyzed by ultrahigh performance liquid chromatography-tandem mass spectroscopy (UPLC-MS/MS) were prepared by removing proteins to recover chemically diverse metabolites. The resulting extract was divided into five fractions: two for analysis by two separate reverse phase (RP)/UPLC-MS/MS methods with positive ion mode electrospray ionization (ESI), one for analysis by RP/UPLC-MS/MS with negative ion mode ESI, one for analysis by HILIC/UPLC-MS/MS with negative ion mode ESI. The sample extract was dried then reconstituted in solvents compatible to each of the four methods. Each reconstitution solvent contained a series of standards at fixed concentrations to ensure injection and chromatographic consistency. Raw data was extracted, peak-identified by comparison to library entries of purified standards or recurrent unknown entities

### Results

*Demographics:* a cohort of 313 patients with ASD with completed clinical data in the Greenwood Genetic Center (GGC, SC, USA) database was considered in order to identify ASD Phenotype 1 as per the association of specific clinical signs and symptoms.

Out of these 313 ASD patients in the GGC database, 90 (28.8%) had at least two well documented measures of head circumference taken in the first 24 months of life by a trained physician. Among these 90 patients, 47 (52.2%) matched with at least 1 primary criterion (i.e. head circumference (HC)).

The families of the 47 patients with at HC>75 were contacted by telephone to inquire about the presence of the second mandatory criteria for ASD Phenotype 1. The GGC failed to establish contact with the families of 5 of the 47 patients (10.6%). Of the remaining 42 patients from which it was possible to collect further clinical information, 21 (50%) satisfied the ASD Phenotype 1 criteria. Overall, with the exclusion of the 5 cases which could not be followed-up, 21 out of 85 patients (24.7%) fit the criteria for ASD phenotype 1 and showed between 3 and 8 of the secondary characteristics.

Among the 21 ASD Phenotype 1 patients, 10 patients were randomly selected for plasma metabolomic profiling. Five typically developing individuals, with no history of neurodevelopment disorder and aged matched, were also selected.

*Metabolomic findings:* There was clear evidence of different levels of metabolites related to pentose and purine metabolism in ASD phenotype 1 plasma (see Figure 1). Plasma levels for each metabolite were measured with UPLC-MS/MS and normalized using block correction method. As illustrated in Figure 1, levels of ribitol, lyxonate and erythritol were significantly higher in plasma from ASD Phenoytpe 1 patients compared to plasma from TD. Levels of urate were significantly lower in plasma from ASD Phenoytpe 1 patients compared to plasma from TD and levels of ribose showed a trend in increasing in plasma from ASD Phenotype 1 patients compared to plasma from TD.

In addition, we used levels of metabolites found to be associated with Rett syndrome, a neurodevelopmental disorder (NDD)(Neul et al. Metabolic Signatures Differentiate Rett Syndrome From Unaffected Siblings. Front Integr. Neurosci. 2020 Feb 25;14:7.) and in ASD Phenotype 1, namely creatinine, 1,5-anhydroglucitol, N-acetylcarnosine and deoxycarnitine, as a control for NDD disease state. Ratio between ASD phenotype 1-specific metabolites and NDD disease state metabolites was computed for all the possible combinations and six ratios were retained as showing discrimination between ASD Phenotype 1 and TD.

As illustrated in Figure 2, ratios between ribitol and creatinine, ribitol and 1,5-anhydroglucitol, ribitol and 5- deoxycarnitine, urate and N-acetylcarnosine, erythritol and 1,5-anhydroglucitol and erythritol and N-acetylcarnosine were all higher in patients with ASD Phenotype 1 compared to TD individuals.

We used Principal Component Analysis (PCA) to determine if the combination of all these ratios together would lead to a clear discrimination between patients with ASD Phenotype 1 and TD individuals. As illustrated in Figure 3, the data points are grouped in two clusters according to their phenotype, i.e ASD phenotype 1 and TD. This result indicates that combining the six ratios together in one analysis leads to a clear differentiation between ASD Phenotype 1 and TD.

### Example 2

Prior to metabolomic characterization of plasma samples from ASD phenotype 1 patients, patients were classified as ASD phenotype 1 or controls as in example 1. In addition, patients symptoms were quantified using the Aberrant Behavior Checklist (ABC) - Second Edition- Community (Kaat et al. Validity of the Aberrant Behavior Checklist in Children with Autism Spectrum Disorder. Journal of Autism and Developmental Disorders. 2014 May; 44:5).

### Description of the ABC scale

The ABC is a questionnaire divided into 5 subscales: Irritability, Lethargy/Social withdrawal, Stereotypic behavior, Hyperactivity, and Inappropriate speech. The ABC checklist is given to a caregiver/informant who provides information for the patients. The higher the score performed on the subscale, the higher the levels of behavioral deficits in the given subcategory of symptoms.

Plasma sample analysis and metabolite quantification was performed in the same way as described in Example 1. The correlation coefficient between the metabolite ratios and the results of each subscale of the ABC questionnaire was obtained for each individual. A correlation was considered positive when the R² was superior to 0.3. Four positive correlations were identified between the ribitol to creatinine ratio and the levels of irritability and hyperactivity and between the ribitol to 1,5-anhydroglucitol ratio and the levels of irritability and hyperactivity.

Figure 4 shows the correlation between levels of ribitol to creatinine ratios and the levels of irritability and hyperactivity measured using the Activities-Specific Balance Confidence (ABC) scale. High scores in the ABC scales relates to aggravated symptoms and lower scores to levels found in TD individuals. The negative correlation between ribitol to creatinine ratios and irritability and hyperactivity levels indicates that high ratios correspond to low degree of behavioral symptoms.

Figure 5 shows the correlation between levels of ribitol to 1,5-anhydroglucitol and the levels of irritability and hyperactivity measured using the Activities-Specific Balance Confidence (ABC) scale. High scores in the ABC scales relates to aggravated symptoms and lower scores to levels found in TD individuals. The negative correlation between ribitol to creatinine ratios and irritability and hyperactivity levels indicates that high ratios correspond to low degree of behavioral symptoms.

These results suggest that in addition to identifying a subpopulation of ASD, namely ASD phenotype 1, the levels of two of these ratios reflect the severity of the symptoms of the disease. It follows that the ratio between these metabolites can be used to monitor the severity of the symptoms, including disease progression, and for monitoring pharmacological or therapeutic treatment efficacy, all by using biological markers specific to ASD patients.

## Claims

1. A method for diagnosing ASD in a subject, comprising
a) providing a sample from a subject;
b) measuring the level of at least one metabolic marker selected from the group consisting of ribitol, lyxonate, erythritol, ribose and urate in said sample; and
c) diagnosing ASD if the level of the at least one metabolic marker measured in step b) is specifically different in comparison to a typically developing control.

2. The method according to claim 1, wherein specifically different means a higher level in case of ribitol, lyxonate, erythritol or ribose and a lower level in case of urate.

3. A method for diagnosing ASD in a subject, comprising
a) providing a sample from a subject;
b) measuring at least one metabolite ratio selected from the group consisting of ribitol/creatinine, ribitol/1,5-anhydroglucitol, ribitol/deoxycarnithine, urate/N-acetylcarnosine, erythritol/1,5-anyhdroglucitol and erythrithol/N-acetylcarnosine in said sample; and
c) diagnosing ASD if the at least one metabolite ratio measured in step b) is increased in comparison to a typically developing control.

4. The method according to claim 3, wherein in step b), 2, 4 or 6 metabolite ratios are measured and averaged and in step c), this average is compared to the average of the respective 2, 4 or 6 metabolite ratios as measured in a sample from a typically developing control.

5. The method according to claim 4, wherein the subject is diagnosed with ASD if the mean value of the ratios shows an increase of at least 15 percent, in comparison to a typically developing controls.

6. The method according to any of claims 1 to 5, wherein in step c), the diagnosis is ASD phenotype 1.

7. A method for monitoring the progression of ASD in a patient suffering from ASD, comprising
a) providing a first sample s₀ from the patient;
b) measuring the level of at least one metabolic marker selected from the group consisting of ribitol, lyxonate, erythritol, ribose and urate in said first sample;
c) providing a second sample s₁ from the patient;
d) measuring the level of the same metabolic marker as in step b) in said second sample; and
e) determining whether the ASD has progressed in the patient by comparing the level obtained in step b) with the one obtained in step d) .

8. The method according to claim 7, wherein in step e) it is determined that the ASD has progressed in the patient if, in case the at least one metabolic marker is ribitol, lyxonate, erythritol or ribose, the level in the second sample is higher than the level in the first sample or if, in case the at least one metabolic marker is urate, the level in the second sample is lower than the level in the first sample.

9. A method for monitoring the progression of ASD in a patient suffering from ASD, comprising
a) providing a first sample s₀ from the patient;
b) measuring at least one metabolite ratio selected from the group consisting of ribitol/creatinine, ribitol/1,5-anhydroglucitol, ribitol/deoxycarnithine, urate/N-acetylcarnosine, erythritol/1,5-anyhdroglucitol and erythrithol/N-acetylcarnosine in said first sample;
c) providing a second sample s₁ from the patient;
d) measuring the same metabolite ratio selected from the group consisting of ribitol/creatinine, ribitol/1,5-anhydroglucitol, ribitol/deoxycarnithine, urate/N-acetylcarnosine, erythritol/1,5-anyhdroglucitol and erythrithol/N-acetylcarnosine as in step b) in said second sample; and
e) determining whether the ASD has progressed in the patient by comparing the ratios measured in step b) with the one obtained in step d).

10. The method according to claim 9, wherein in step e) it is determined that the ASD has progressed in the patient if the ratio measured in the second sample is higher than the corresponding ratio measured in the first sample .

11. The method according to claim 9 or 10, wherein 2, 4 or 6 ratios for each sample are measured, the mean value of these ratios is calculated for each sample and these mean values are used in step e) instead of the ratios for determining whether the ASD has progressed.

12. The method according to any of claims 7 to 11, wherein the patients suffers from ASD phenotype 1.

13. A method for determining efficacy of an ASD treatment in an ASD patient, comprising:
a) providing samples from the patient obtained before and after application of the treatment; and
b) measuring at least one metabolite ratios selected from the group consisting of ribitol/creatinine, ribito/1,5-anhydroglucitol, ribitol/deoxycarnithine, urate/N-acetylcarnosine, erythritol/1,5-anyhdroglucitol and erythrithol/N-acetylcarnosine in the samples of step a).

14. The method according to claim 13, wherein the treatment is determined effective if the ratio measured in the sample obtained after application of the treatment is lower than the ratio measured in the sample obtained before application of the treatment.

15. The method according to any of claims 1 to 14, wherein the sample is a blood sample, preferably a plasma sample.
